# EUROPÄISCHE PATENTSCHRIFT

(11) **EP 0 703 026 B1**
(45) Veröffentlichungstag und Bekanntmachung des Hinweises auf die Patenterteilung: **20.10.1999**
(21) Anmeldenummer: 95113276.0
(22) Anmeldetag: 24.08.1995
(51) Int. Cl.: B22D 2/00, C21C 5/46, C25C 3/20, G01N 1/12, G01N 25/04, G01K 13/12

(54) **Sensoranordnung zur Temperaturmessung**
Sensor arrangement for temperature measurement
Dispositif détecteur pour mesurer la température

(30) Priorität: 21.09.1994 DE 4433685
(43) Veröffentlichungstag der Anmeldung: 27.03.1996
(73) Patentinhaber: Heraeus Electro-Nite International N.V., 3530 Houthalen (BE)
(72) Erfinder: Verstreken, Paul Clement, B-3500 Hasselt (BE); Aegten, Jozef Theodoor, B-3950 Bocholt (BE)
(74) Vertreter: Kühn, Hans-Christian

(56) Entgegenhaltungen:
- DE-A- 2 225 766
- DE-A- 2 844 417
- DE-B- 2 730 813
- US-A- 3 643 509
- US-A- 4 842 417
- GIESSEREI, Bd. 76, Nr. 2, 23.Januar 1989 DÜSSELDORF DE, Seiten 47-53, R. DÖPP 'Beitrag zur Beurteilung .....'
- DATABASE WPI Section EI, Week 8811 Derwent Publications Ltd., London, GB; Class S03, AN 88-075816 & SU-A-1 326 974 (KUIBYSHEV POLY) , 30.Juli 1987

## Beschreibung

Die Erfindung betrifft eine Sensoranordnung zur Temperaturmessung von Schmelzen mit einem Behälter, der mindestens einen streifen- oder drahtförmigen Träger und an seiner Oberseite eine Öffnung aufweist, und mit einem in dem Behälter angeordneten Thermoelement. Die Erfindung betrifft auch eine Temperatureinrichtung und ein Verfahren zur Messung der Liquidustemperatur von Kryolithschmelzen.

Derartige Sensoranordnungen werden beispielsweise zur Bestimmung der Liquidustemperatur von Schmelzen verwendet, wobei die Abkühlungskurve der in den Behälter eingefüllten Schmelze bestimmt wird. Aus der Liquidustemperatur können Informationen über die Zusammensetzung der Schmelze erhalten werden. Eine bekannte Vorrichtung der eingangs genannten Art für die Messung der Liquidusmessung von Kryolithschmelzen weist einen Graphittiegel zur Probennahme auf, in dem ein Thermoelement angeordnet ist. Der Graphittiegel ist mittels eines Metallstabes an einer Halterung befestigt. Der Graphittiegel wird zur Probennahme in die Kryolithschmelze getaucht und mit einem Schmelzenvolumen von etwa 3 cm³nach Erreichen des thermischen Gleichgewichtes aus der Schmelze herausgezogen. Danach wird die Abkühlungskurve registriert und daraus die Liquidustemperatur bestimmt. Die mit dieser Meßvorrichtung gewonnenen Werte für die Liquidustemperatur weisen eine Schwankung von mehreren Grad auf, sind also sehr ungenau, so daß die Meßergebnisse in der Praxis nicht zuverlässig benutzt werden können.

Eine andere Vorrichtung zur Temperaturmessung ist aus US 3,643,509 bekannt. Mit der hier beschriebenen Vorrichtung ist es möglich, Liquidusmessungen in Stahl vorzunehmen. Dabei ist ein Thermoelement in einem U-förmigen Quarzröhrchen innerhalb eines Behälters aus Quarz angeordnet. Der Behälter ist in üblicher Art an der Spitze eines Meßkopfes angeordnet und weist einige seitliche Einlauföffnungen für die Stahlschmelze auf. Diese Vorrichtung wird nach Eintauchen in die Stahlschmelze zur Messung der Badtemperatur und nach Herausziehen aus der Stahlschmelze zur Messung der Liquidustemperatur verwendet. Derartige Anordnungen sind allerdings für Schmelzen mit beispielsweise niedriger Schmelzwärme und schlechter Wärmeleitfähigkeit, wie Kryolithschmelzen, nicht einsetzbar.

Aufgabe der vorliegenden Erfindung ist es, eine Sensoranordnung der eingangs genannten Art zu schaffen, mit der die exakte Messung der Liquidustemperatur von Kryolithschmelzen ermöglicht, und die gleichzeitig kostengünstig herstellbar ist. Aufgabe der Erfindung ist es weiterhin, eine Temperaturmeßeinrichtung und ein Verfahren zur Messung der Liquidustemperatur von Kryolithschmelzen bereitzustellen, mit dem Meßergebnisse mit einer hohen Genauigkeit reproduzierbar erzielt werden können.

Diese Aufgabe wird für die eingangs beschriebene Sensoranordnung dadurch gelöst, daß der Behälter aus Metall gebildet ist und daß der mindestens eine Träger mit einem Vibrator starr verbunden ist. Ein derartiger Behälter hat eine relativ geringe Wärmekapazität und eine hohe Wärmeleitfähigkeit, so daß der Behälter nur eine sehr geringe Wärmemenge aus der Kryolithschmelze aufnehmen kann. Dabei ist es zweckmäßig, daß der Behälter eine Wandstärke von weniger als 0,5 mm, insbesondere weniger als 0,2 mm aufweist und daß als Material für den Behälter vorzugsweise Kupfer verwendet wird.

Bei Eintauchen einer kalten Anordnung in Kryolithschmelze erstarrt diese sofort an den Bauteilen mit niedrigerer Temperatur, bei Erreichen eines Gleichgewichtszustandes wird dieses erstarrte Kryolith jedoch wieder schmelzen. Dieses Wiederaufschmelzen erfolgt in einem dünnwandigen Behälter mit einer hohen Wärmeleitfähigkeit am schnellsten, da zum einen nur eine sehr geringe Wärmemenge durch den Behälter aufgenommen werden kann, zum anderen wird der Behälter durch eine hohe Wärmeleitfähigkeit sehr schnell aufgeheizt.

Für eine exakte Messung ist es vorteilhaft, daß der Behälter eine gewellte Oberfläche aufweist. Dadurch wird die Oberfläche der erstarrenden Kryolithschmelze erhöht; die Erstarrung findet nach dem Herausziehen der Sensoranordnung aus der Schmelze zuerst im Bereich dieser Oberfläche statt und setzt sich dann gleichmäßig ins Innere der Schmelze fort.

Zweckmäßig ist es, daß das Thermoelement in einem Quarzrohr, insbesondere in einem einseitig geschlossenen Quarzrohr angeordnet ist, welches eine nichtoxidische Schutzschicht aufweist. Ein derartiges Thermoelement ist beständig gegen Kryolithschmelze und kann mit einem kleinen Volumen ausgeführt werden, so daß nur eine sehr geringfügige Wärmeableitung über das Thermoelement erfolgt. Zweckmäßig ist es, die Schutzschicht aus einem temperaturbeständigen Metall oder aus einer nichtoxidischen Keramik auszubilden, um die Resistenz gegenüber der Kryolithschmelze zu erhöhen. Für eine exakte Aufzeichnung der Abkühlungskurve ist es vorteilhaft, daß das Thermoelement etwa in der Mitte des Behälters angeordnet ist.

Um eine stabile Handhabung der Sensoranordnung zu sichern, ist es zweckmäßig, daß die Träger aus Metalldrähten gebildet sind, da diese eine hohe Resistenz gegenüber der Kryolithschmelze aufweisen.

Weiterhin kann es zweckmäßig sein, daß die Innenoberfläche des Behälters eine Rauhigkeit größer als 1,25 µm, vorzugsweise zwischen 2,5 µm und 15 µm aufweist.

Die Aufgabe wird für eine Temperaturmeßeinrichtung mit einer Sensoranordnung der oben beschriebenen Art dadurch gelöst, daß der mindestens eine Träger an seiner dem Behälter abgewandten Seite in einer Hülse gehaltert ist und daß die Hülse mit einer Halterung lösbar verbunden ist. Es ist jedoch auch möglich, die Hülse unlösbar mit der Halterung zu verbinden. Eine derartige Anordnung gewährleistet eine hohe Stabilität und eine einfache Handhabung der Einrichtung während der Durchführung von Messungen. Zweckmäßig ist es, die Hülse im wesentlichen aus feuerfestem Material zu bilden, da dann die Länge der Träger verkürzt werden kann, ohne daß die Hülse während des Eintauchens des Behälters in die Schmelze durch die aus der Schmelze aufsteigende Wärme zerstört wird. Die Halterung kann beispielsweise als eine in der Metallurgie übliche Lanze oder Papprohr ausgeführt sein.

Vorteilhaft ist es, daß das Thermoelement in der Hülse gehaltert und mit einem Verbindungsstück der Hülse leitend verbunden ist und daß das Verbindungsstück mit Signalleitungen der Halterung in Kontakt steht. Dadurch sind der Behälter zur Entnahme der Schmelzenprobe und das Thermoelement zu einer Einheit zusammengefaßt, die aus der Halterung entfernt und gegen eine neue Einheit nach der Messung ausgetauscht werden kann. Die Signalleitungen, die das elektrische Signal des Thermoelementes an eine Auswerteeinheit weiterleiten, können innerhalb der Halterung geführt sein und sind so vor Beschädigung geschützt.

Für die Realisierung einer homogenen Erstarrung der Schmelze ist es vorteilhaft, daß die Halterung mit einem Vibrator starr verbunden ist.

Erfindungsgemäß wird die Aufgabe für ein Verfahren zur Messung der Liquidustemperatur von Kryolithschmelzen dadurch gelöst, daß während der Messung der Abkühlungskurve der Kryolithschmelze in einem Behälter dieser Behälter vibriert. Dadurch wird eine homogene Erstarrung der Kryolithschmelze, beginnend an der Behälteroberfläche, bewirkt. Durch die Vibration der Kryolithschmelze während des Abkühlens werden Unterkühlungseffekte in der Schmelze vermieden. Die Vibrationsfrequenz beträgt etwa 20 bis 1000 Hz, vorzugsweise 150 bis 400 Hz, die Vibrationsamplitude beträgt etwa 0,01 bis 0,5 mm, vorzugsweise 0,08 bis 0,15 mm.

Nachfolgend wird die Erfindung an einem Ausführungsbeispiel anhand einer Zeichnung näher erläutert. In der Zeichnung zeigt:
Figur 1 eine schematische Darstellung der Sensoranordnung,
Figur 2 eine bevorzugte Ausführungsform des Behälters und
Figur 3 eine schematische Darstellung der Temperaturmeßeinrichtung einschließlich der Halterung.

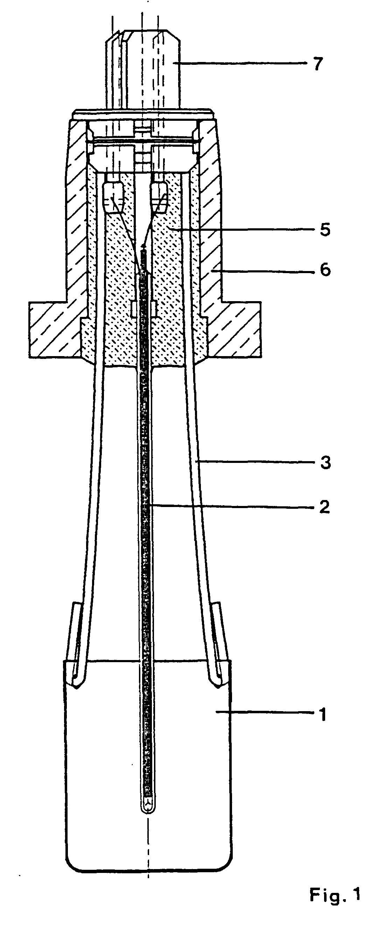

Figur 1 zeigt eine Sensoranordnung, bei der in einem Behälter 1 ein Thermoelement 2 angeordnet ist. Der Behälter 1 ist aus Kupfer und weist eine Wandstärke von 0,1 mm auf. Die Drähte des Thermoelementes 2 sind innerhalb eines Quarzröhrchens angeordnet, das an seinem in den Behälter 1 hineinragenden Ende geschlossen ist. Das Quarzröhrchen weist eine Beschichtung aus Metall oder einer nichtoxidischen Keramik, beispielsweise TiB₂ TiN oder BN auf. Diese Schicht kann mittels Flammspritzen, Plasmaspritzen oder durch Aufdampen aufgebracht werden. Auch eine Tauchbeschichtung oder ein ähnliches Beschichtungsverfahren sind möglich.

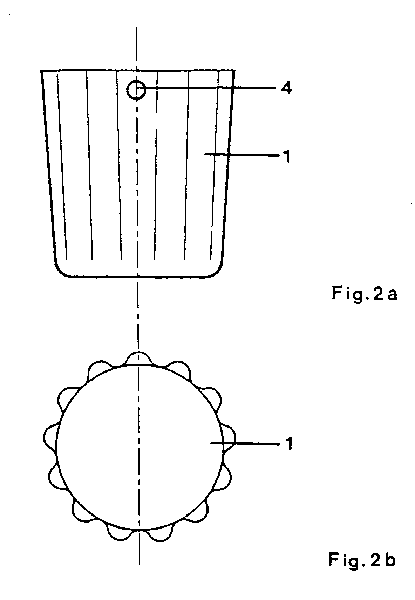

Der rotationssymmetrische Behälter 1 ist an drei aus Metalldrähten gebildeten Trägern 3 befestigt. Die Träger 3 können beispielsweise mit dem Behälter 1 verschweißt sein. Als Material für die Träger 3 wird Stahl mit einem Druchmesser von 1 mm verwendet. Der Behälter 1 ist detailliert in Figur 2 dargestellt. Dabei zeigt Figur 2a eine Seitenansicht des Behälters 1 mit einer Öffnung 4, in der ein Träger 3 befestigt wird. Figur 2b zeigt eine Draufsicht des Behälters 1; hierbei ist die gewellte Umfangsfläche deutlich erkennbar. Der Behälter 1 weist innen eine Oberflächenrauhigkeit von etwa 2,5 bis 15 µm auf.

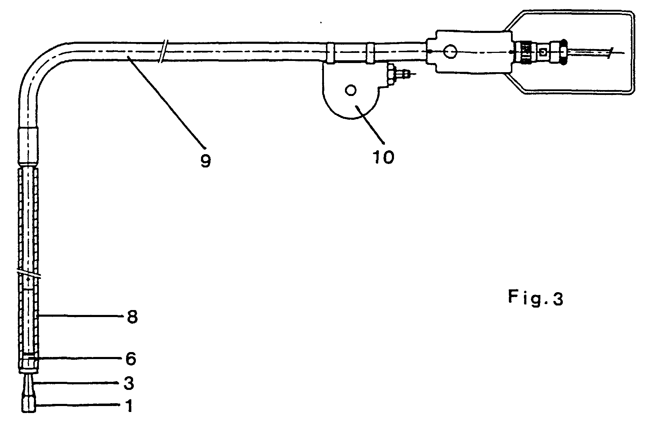

Die Träger 3 und das Thermoelement 2 sind mittels Zement 5 in einer Hülse 6 aus einem feuerfesten Material, beispielsweise Kordierit befestigt. In der Hülse 6 sind die Thermodrähte des Thermoelementes 2 mit Kontakten des Verbindungsstückes 7 verbunden. Die Hülse 6 ist, wie in Figur 3 gezeigt, in dem Ende der Halterung 8 angeordnet. Dort sind die Kontakte des Verbindungsstückes 7 leitend mit Signalleitungen verbunden, die durch die Halterung 8 hindurch geführt sind und über die Lanze 9 an einer nachgeschalteten Meß- und Auswerteelektronik angeschlossen werden können. Mit der Halterung 8 und der Lanze 9 ist starr ein Vibrator 10 verbunden, der den Behälter 1 mit der zu messenden Kryolithschmelze während der Aufnahme der Abkühlungskurve in Schwingungen versetzt.

Die Frequenz der Schwingungen kann in einem sehr breiten Bereich gewählt werden, sie liegt jedoch vorzugsweise zwischen 150 und 400 Hz, um Unterkühlungseffekte der abkühlenden Schmelze zu vermeiden. Die Amplitude der Schwingungen beträgt etwa 0,08 bis 0,15 mm.

## Patentansprüche

1. Sensoranordnung zur Temperaturmessung von Schmelzen mit einem Behälter, der mindestens einen streifen- oder drahtförmigen Träger und an seiner Oberseite eine Öffnung aufweist, und mit einem in dem Behälter angeordneten Thermoelement, dadurch gekennzeichnet, daß der Behälter (1) aus Metall gebildet ist und daß der mindestens eine Träger (3) mit einem Vibrator (10) starr verbunden ist.

2. Sensoranordnung nach Anspruch 1, dadurch gekennzeichnet, daß der Behälter (1) eine Wandstärke von weniger als 0,5 mm aufweist.

3. Sensoranordnung nach Anspruch 2, dadurch gekennzeichnet, daß die Wandstärke geringer ist als 0,2 mm.

4. Sensoranordnung nach einem der Ansprüche 1 bis 3, dadurch gekennzeichnet, daß der Behälter (1) aus Kupfer gebildet ist.

5. Sensoranordnung nach einem der Ansprüche 1 bis 4, dadurch gekennzeichnet, daß der Behälter (1) eine gewellte Oberfläche aufweist.

6. Sensoranordnung nach einem der Ansprüche 1 bis 5, dadurch gekennzeichnet, daß das Thermoelement (2) in einem Quarzglasrohr angeordnet ist, das eine nichtoxidische Schutzschicht aufweist.

7. Sensoranordnung nach Anspruch 6, dadurch gekennzeichnet, daß das Thermoelement (2) in einem einseitig geschlossenen Quarzglasrohr angeordnet ist.

8. Sensoranordnung nach Anspruch 6 oder 7, dadurch gekennzeichnet, daß die Schutzschicht aus einem temperaturbeständigen Metall gebildet ist.

9. Sensoranordnung nach Anspruch 6 oder 7, dadurch gekennzeichnet, daß die Schutzschicht aus einer nichtoxidischen Keramik gebildet ist.

10. Sensoranordnung nach einem der Ansprüche 1 bis 9, dadurch gekennzeichnet, daß das Thermoelement (2) etwa in der Mitte des Behälters (1) angeordnet ist.

11. Sensoranordnung nach einem der Ansprüche 1 bis 10, dadurch gekennzeichnet, daß die Träger (3) aus Metalldrähten gebildet sind.

12. Sensoranordnung nach einem der Ansprüche 1 bis 10, dadurch gekennzeichnet, daß die Innenoberfläche des Behälters (1) eine Rauhigkeit größer als 1,25 µm aufweist.

13. Sensoranordnung nach Anspruch 12, dadurch gekennzeichnet, daß die Innenoberfläche des Behälters (1) eine Rauhigkeit zwischen 2,5 µm und 15 µm aufweist.

14. Temperaturmeßeinrichtung mit einer Sensoranordnung nach einem der Ansprüche 1 bis 13, dadurch gekennzeichnet, daß der mindestens eine Träger (3) an seiner dem Behälter (1) abgewandten Seite in einer Hülse (6) gehaltert ist und daß die Hülse (6) mit einer Halterung (8) verbunden ist.

15. Temperaturmeßeinrichtung nach Anspruch 14, dadurch gekennzeichnet, daß die Hülse (6) im wesentlichen aus feuerfestem Material gebildet ist.

16. Temperaturmeßeinrichtung nach Anspruch 14 oder 15, dadurch gekennzeichnet, daß das Thermoelement (2) in der Hülse (6) gehaltert und mit einem Verbindungsstück (7) der Hülse (6) leitend verbunden ist und daß das Verbindungsstück (7) mit Signalleitungen der Halterung (8) in Kontakt steht.

17. Temperaturmeßeinrichtung nach einem der Ansprüche 14 bis 16, dadurch gekennzeichnet, daß die Halterung (8) mit einem Vibrator (10) starr verbunden ist.

18. Verfahren zur Messung der Liquidustemperatur von Kryolithschmelzen in einem Behälter, wobei die Abkühlungskurve der Kryolithschmelze gemessen wird, dadurch gekennzeichnet, daß der Behälter (1) während des Abkühlens der Kryolithschmelze vibriert wird.

19. Verfahren nach Anspruch 18, dadurch gekennzeichnet, daß die Vibrationsfrequenz 20 bis 1000 Hz, vorzugsweise 150 bis 400 Hz beträgt.

20. Verfahren nach Anspruch 18 oder 19, dadurch gekennzeichnet, daß die Vibrationsamplitude etwa 0,01 bis 0,5 mm, vorzugsweise 0,08 bis 0,15 mm beträgt.

## Claims

1. A sensor arrangement for temperature measurement of melts with a container which has at least one carrier in strip or wire form and has an opening on its upper face, and with a thermocouple arranged in the container, characterised in that the container (1) is formed from metal and that the at least one carrier (3) is rigidly connected with a vibrator (10).

2. The sensor arrangement according to Claim 1, characterised in that the container (1) has a wall thickness of less than 0.5 mm.

3. The sensor arrangement according to Claim 2, characterised in that the wall thickness is less than 0.2 mm.

4. The sensor arrangement according to one of Claims 1 to 3, characterised in that the container (1) is formed from copper.

5. The sensor arrangement according to one of Claims 1 to 4, characterised in that the container (1) has a corrugated surface.

6. The sensor arrangement according to one of Claims 1 to 5, characterised in that the thermocouple (2) is arranged in a quartz glass tube which has a non-oxidic protective layer.

7. The sensor arrangement according to Claim 6, characterised in that the thermocouple (2) is arranged in a quartz glass tube which is closed on one side.

8. The sensor arrangement according to Claim 6 or 7, characterised in that the protective layer is formed from a temperature-resistant metal.

9. The sensor arrangement according to Claim 6 or 7, characterised in that the protective layer is formed from a non-oxidic ceramic material.

10. The sensor arrangement according to one of Claims 1 to 9, characterised in that the thermocouple (2) is arranged approximately in the centre of the container (1).

11. The sensor arrangement according to one of Claims 1 to 10, characterised in that the carriers (3) are formed from metal wires.

12. The sensor arrangement according to one of Claims 1 to 10, characterised in that the inner surface of the container (1) has a roughness greater than 1.25 µm.

13. The sensor arrangement according to Claim 12, characterised in that the inner surface of the container (1) has a roughness between 2.5 µm and 15 µm.

14. A temperature measurement device with a sensor arrangement according to one of Claims 1 to 13, characterised, in that the at least one carrier (3) is held on its side facing away from the container (1) in a casing (6) and that the casing (6) is connected with a mounting (8).

15. The temperature measurement device according to Claim 14, characterised in that the casing (6) is formed substantially from fireproof material.

16. The temperature measurement device according to Claim 14 or 15, characterised in that the thermocouple (2) is held in the casing (6) and is connected in a conducting manner with a connecting piece (7) of the casing (6) and that the connecting piece (7) is in contact with signal lines of the mounting (8).

17. The temperature measurement device according to one of Claims 14 to 16, characterised in that the mounting (8) is rigidly connected with a vibrator (10).

18. A method for measurement of the liquid temperature of cryolite melts in a container, whereby the cooling curve of the cryolith melt is measured, characterised in that the container (1) is vibrated during the cooling of the cryolith melt.

19. The method according to Claim 18, characterised in that the vibration frequency amounts to 20 to 1000 Hz, preferably 150 to 400 Hz.

20. The method according to Claim 18 or 19, characterised in that the vibration amplitude amounts to approximately 0.01 to 0.5 mm, preferably 0.08 to 0.15 mm.

## Revendications

1. Dispositif détecteur pour mesurer la température de bains de fusion, comportant un récipient qui présente au moins un pied en forme de lame ou de fil raide et présente à sa partie supérieure une ouverture, ainsi qu'un thermocouple disposé dans le récipient, caractérisé par le fait que le récipient (1) est formé de métal et que le pied, (3) dont il y a au moins un, est solidarisé à un vibrateur (10).

2. Dispositif détecteur selon la revendication 1, caractérisé par le fait que le récipient (1) présente une épaisseur de paroi inférieure à 0,5 mm.

3. Dispositif détecteur selon la revendication 2, caractérisé par le fait que l'épaisseur de paroi est inférieure à 0,2 mm.

4. Dispositif détecteur selon l'une des revendications 1 à 3, caractérisé par le fait que le récipient (1) est en cuivre.

5. Dispositif détecteur selon l'une des revendications 1 à 4, caractérisé par le fait que le récipient (1) présente une surface ondulée.

6. Dispositif détecteur selon l'une des revendications 1 à 5, caractérisé par le fait que le thermocouple (2) est disposé dans un tube de verre de quartz qui présente une couche de protection ne résultant pas de l'oxydation.

7. Dispositif détecteur selon la revendication 6, caractérisé par le fait que le thermocouple (2) est disposé dans un tube de verre de quartz fermé d'un côté.

8. Dispositif détecteur selon la revendication 6 ou 7, caractérisé par le fait que la couche de protection est formée d'un métal résistant à haute température.

9. Dispositif détecteur selon la revendication 6 ou 7, caractérisé par le fait que la couche de protection est formée d'une céramique ne résultant pas de l'oxydation.

10. Dispositif détecteur selon l'une des revendications 1 à 9, caractérisé par le fait que le thermocouple (2) est disposé à peu près au milieu du récipient (1).

11. Dispositif détecteur selon l'une des revendications 1 à 10, caractérisé par le fait que les pieds (3) sont formés de fils métalliques.

12. Dispositif détecteur selon l'une des revendications 1 à 10, caractérisé par le fait que la surface intérieure du récipient (1) présente une rugosité supérieure à 1,25 µm.

13. Dispositif détecteur selon la revendication 12, caractérisé par le fait que la surface intérieure du récipient (1) présente une rugosité valant entre 2,5 µm et 15 µm.

14. Appareil de mesure de la température comportant un dispositif détecteur selon l'une des revendications 1 à 13, caractérisé par le fait que le pied (3), dont il y a au moins un, est tenu dans un fourreau (6) du côté opposé au récipient (1) et que le fourreau (6) est relié à un support (8).

15. Appareil de mesure de la température selon la revendication 14, caractérisé par le fait que le fourreau (6) est essentiellement formé d'un matériau réfractaire.

16. Appareil de mesure de la température selon la revendication 14 ou 15, caractérisé par le fait que le thermocouple (2) est tenu dans un fourreau (6) et qu'il est relié, avec conduction, à un raccord (7) du fourreau (6) et que le raccord (7) est en contact avec des lignes de signaux du support (8).

17. Appareil de mesure de la température selon l'une des revendications 14 à 16, caractérisé par le fait que le support (8) est solidarisé à un vibrateur (10).

18. Procédé de mesure de la température du liquidus de bains de fusion de cryolithe dans un récipient, dans lequel on mesure la courbe de refroidissement du bain de fusion de cryolithe, caractérisé par le fait que pendant le refroidissement du bain de fusion de cryolithe on fait vibrer le récipient (1).

19. Procédé selon la revendication 8, caractérisé par le fait que la fréquence de vibration vaut 20 à 1000 Hz, de préférence de 150 à 400 Hz.

20. Procédé selon la revendication 18 ou 19, caractérisé par le fait que l'amplitude de vibration vaut environ 0,01 à 0,5 mm, de préférence 0,08 à 0,15 mm.
